# EUROPÄISCHE PATENTSCHRIFT

(11) **EP 0 661 020 B1**
(45) Veröffentlichungstag und Bekanntmachung des Hinweises auf die Patenterteilung: **16.05.2001**
(21) Anmeldenummer: 94119096.9
(22) Anmeldetag: 03.12.1994
(51) Int. Cl.: A61B 3/13, G02B 21/08

(54) **Schaltbare Beleuchtungseinrichtung für ein Operationsmikroskop**
Switched illumination device for an operation microscope
Dispositif d'éclairage commutable pour un microscope chirurgical

(30) Priorität: 28.12.1993 DE 4344770
(43) Veröffentlichungstag der Anmeldung: 05.07.1995
(73) Patentinhaber: Leica Mikroskopie Systeme AG, 9435 Heerbrugg (CH)
(72) Erfinder: Geschwentner, Otto, CH-9346 Balgach (CH)
(74) Vertreter: Stamer, Harald

(56) Entgegenhaltungen:
- DE-A- 4 214 445
- DE-U- 9 103 433
- DE-U- 9 306 412

## Beschreibung

Die Erfindung betrifft eine schaltbare Beleuchtungseinrichtung für ein Operationsmikroskop in der Augenchirurgie, gemäß den Oberbegriffen der Patentansprüche 1, 5 und 7.

Beim Einsatz von Operationsmikroskopen in der Augenchirurgie, insbesondere bei einer Katarakt-Extraktion, soll der Winkel zwischen der Beleuchtungsachse und der Beobachtungsachse des Mikroskops möglichst klein gehalten werden. Der Vorteil dieser Beleuchtungsart ist darin zu sehen, daß die senkrecht auf das Auge fallenden Lichtstrahlen von der Netzhaut diffus reflektiert werden und dadurch die Linsenkapsel des Auges in einem rötlichen Durchlicht erscheint. Dieser Effekt wird auch als "Red-Reflex" bezeichnet. Die Qualität dieses Red-Reflexes ist in der Katarakt-Extraktion von entscheidender Bedeutung. Bei dieser Operation müssen nach dem Entfernen der Augenlinse sämtliche Gewebereste vom Auge entfernt werden. Dies gelingt nur dann, wenn diese Gewebereste ausreichend optisch kontrastiert dargestellt werden.

Im Verlauf der Operation ist jedoch nicht nur die Red-Reflex-Darstellung erforderlich, sondern es muß natürlich auch eine herkömmliche Beleuchtung, vorzugsweise eine Köhler'sche Beleuchtung, gewährleistet sein, um das Operationsfeld auszuleuchten.

In der DE 92 17 517 U1 wird ein Operationsmikroskop mit einer Beleuchtungseinrichtung beschrieben, mit der eine Red-Reflex-Darstellung der Linsenkapsel ermöglicht wird. Zur Kontraststeigerung in Abhängigkeit von einer eventuell vorliegenden Fehlsichtigkeit des Patienten wird eine daran angepaßte zusätzliche Linse zwischen dem Hauptobjektiv und nahe dem Patientenauge in den Strahlengang eingeschwenkt. Die in dieser Schrift beschriebene Beleuchtungseinrichtung ist durch den Nachteil behaftet, daß durch das Einschwenken einer zusätzlichen Linse zwischen Operationsstelle und Hauptobjektiv der Operationsraum verkleinert und damit die Bewegungsfreiheit des Operateurs behindert wird. Auch ist durch den komplizierten mechanischen Aufbau kein schnelles und unkompliziertes Umschalten zwischen der Beleuchtung des Operationsfeldes und der optimalen Erzeugung des Red-Reflexes gegeben.

Aus der DE 40 28 605 A1 ist eine Red-Reflex-Beleuchtungseinrichtung für ein Operationsmikroskop bekannt. Der Red-Reflex wird dadurch erreicht, das aus dem Beleuchtungsstrahlenbündel über speziell ausgestaltete Umlenkelemente nur bestimmte, achsnahe Beleuchtungsstrahlen in Richtung des Hauptobjektivs reflektiert werden. Durch diese Ausblendung von Beleuchtungsstrahlen muß bei dieser Einrichtung ein hoher Lichtverlust in Kauf genommen werden.

Aus der US 4 657 357 ist eine Beleuchtungseinrichtung für ein Operationsmikroskop bekannt, bei der die Beleuchtungsstrahlen über Prismen achsnah durch das Hauptobjektiv geführt werden. Dazu ist es hier jedoch notwendig, das Hauptobjektiv mit aufwendig herzustellenden Ausnehmungen zu versehen.

Aus der DE 42 14 445 A1 ist ebenfalls eine Red-Reflex-Beleuchtungseinrichtung für ein Operationsmikroskop bekannt. Die Beleuchtung erfolgt hier über einen Lichtleiter und eine Blende. Der Red-Reflex wird dadurch erreicht, das aus dem Beleuchtungsstrahlenbündel über speziell ausgestaltete Umlenkelemente nur Beleuchtungsstrahlen in Richtung des Hauptobjektivs reflektiert werden. Die wirksame Breite des Umlenkelements kann variiert werden, um einen homogenen Intensitätsverlauf in Abhängigkeit von der jeweiligen Vergrößerung der Mikroskops und des Patientenauges zu erreichen.

Bei den bekannten Operationsmikroskopen für die Augenchirurgie erscheint die Pupille im Beobachtungsstrahlengang rot, wenn sich die Strahlenkegel der Beleuchtung und die der Beobachtung überschneiden. Die Beobachtung des Red-Reflexes wird jedoch durch die Helligkeit des Objektes außerhalb der Pupille und durch störende Reflexionen des Bildes der Lampenwendel an der Netzhaut des Patienten erschwert.

Es ist daher Aufgabe der vorliegenden Erfindung, die Beleuchtungseinrichtung eines Operationsmikroskops mit einfachen schaltbaren Mitteln auszustatten, um sowohl eine Köhler'sche Beleuchtung als auch durch Umschalten eine optimierte Red-Reflex-Beleuchtung oder eine Kombination beider Beleuchtungen zu realisieren.

Diese Aufgabe wird erfindungsgemäß durch die kennzeichnenden Merkmale der Patentansprüche 1, 5 und 7 gelöst. Vorteilhafte Weiterbildungen der Erfindung sind Gegenstand der Unteransprüche.

Durch Versuche hat es sich herausgestellt, daß sich ein optimierter Red-Reflex einstellt, wenn nicht die Leuchtfeldblende bzw. deren konjugierte Ebene in die zu betrachtende Objektebene abgebildet wird, sondern das Bild der Lampenwendel der Lichtquelle. Zusätzlich wird bei dieser Verlagerung der Abbildung in vorteilhafter Weise erreicht, daß das Objekt außerhalb der Pupille nicht beleuchtet wird.

Ferner wird durch diese spezielle Abbildung kein Bild der Lampenwendel auf der Netzhaut des Auges erzeugt und somit die spezifische Belastung der Netzhaut bei derartigen Operationen minimiert.

Die erfindungsgemäße schaltbare Beleuchtungseinrichtung für Operationsmikroskope wird nachfolgend an Hand der schematischen Zeichnungen in mehreren Ausführungsbeispielen näher erläutert.

In den Zeichnungen zeigen:
- Fig. 1: den Beleuchtungsstrahlengang in einer Schnittdarstellung
- Fig. 2: den Beleuchtungsstrahlengang in einer Aufsicht mit einer zusätzlichen und auf einem Schieber angeordneten Lichtquelle
- Fig. 3: ein Ausführungsbeispiel gemäß der Figur 2 mit einer Negativlinse anstelle der Lichtquelle
- Fig. 4: ein weiteres Ausführungsbeispiel mit einer auf einem Schieber angeordneten Positivlinse
- Fig. 5: ein Ausführungsbeispiel mit einer zusätzlichen Lichtquelle
- Fig. 6: ein Ausführungsbeispiel mit einem Lichtleiter und einer gemeinsamen Lichtquelle
- Fig. 7: ein Ausführungsbeispiel mit einer extern angeordneten Lichtquelle

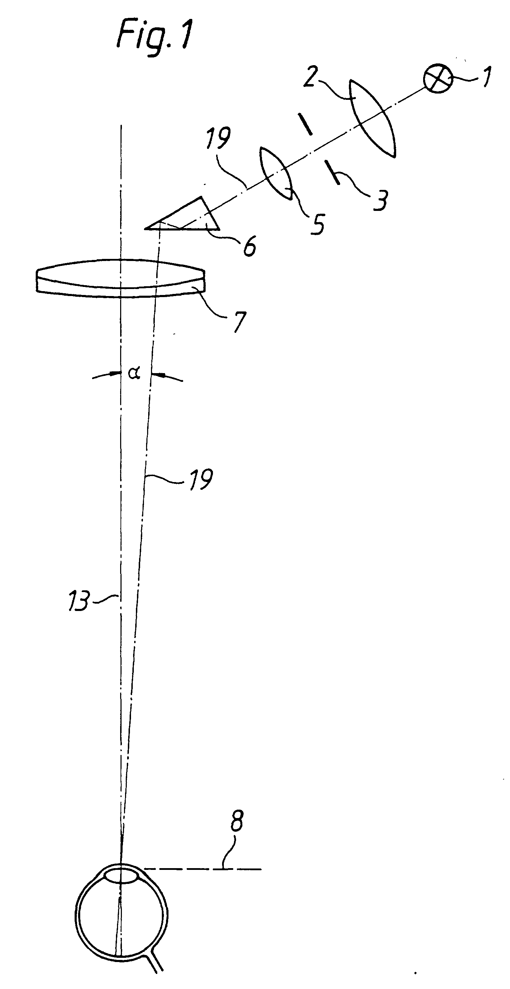

Die Figur 1 zeigt eine Schnittdarstellung durch den Beleuchtungsstrahlengang 19 eines bekannten Mikroskops, bei dem das Licht einer Lichtquelle 1 über ein optisches System mit einem Kollektorlinsensystem 2, einer Leuchtfeldblende 3, einem Umlenkprisma 6, einer weiteren Linse 5 und einem Hauptobjektiv 7 in eine Objektebene 8, geführt wird. Der dargestellte Strahlengang 19 ist als Köhler'scher Beleuchtungsstrahlengang ausgebildet. Dabei wird die Leuchtfeldblende 3 über das nachgeordnete optische System 5, 7 in die Objektebene 8 abgebildet. Über einen Beobachtungsstrahlengang 13 kann das Objekt bzw. die Objektebene mit dem nicht näher dargestellten Mikroskop als helles Bild beobachtet werden.

Die Beleuchtungsstrahlen gelangen durch die Objektebene 8 auch auf die Netzhaut des Auges und erzeugen durch Streuung und Reflexion üblicherweise einen relativ schwachen und häufig ungleichmäßig leuchtenden Red-Reflex.

Wie bereits erwähnt, beeinflußt der Winkel (alpha) zwischen dem Beobachtungsstrahlengang 13 und dem Beleuchtungsstrahlengang 19 die Intensität des Red-Reflexes.

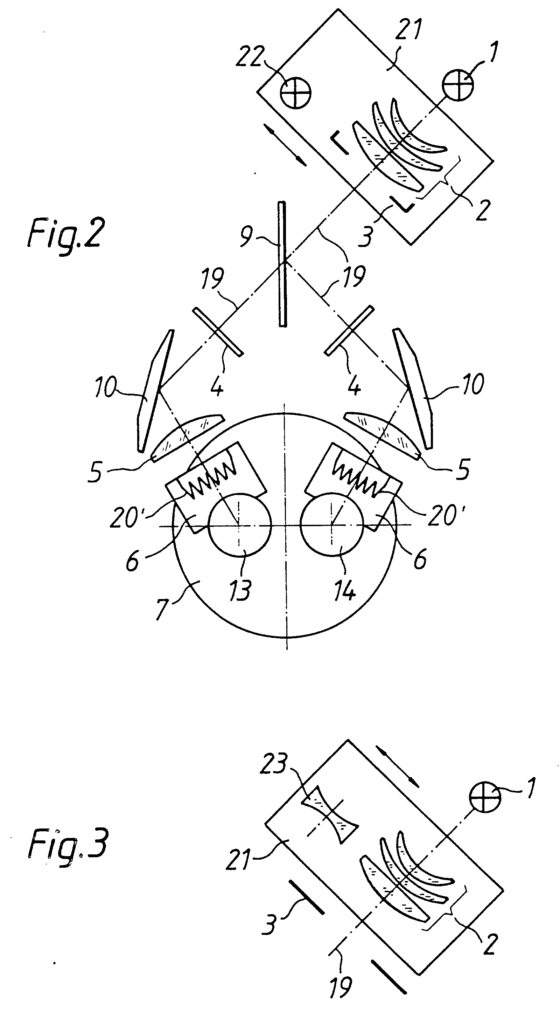

Die Figur 2 zeigt den Beleuchtungsstrahlengang 19 gemäß der Figur 1 in einer Aufsicht. Die Leuchtfeldblende 3 wird zur Erzeugung einer stereoskopischen Beleuchtung über einen Strahlenteiler 9, im Beleuchtungsstrahlengang 19 angeordnete Filter 4, Kaltlichtspiegel 10, Linsen 5 und Umlenkprismen 6 durch das Hauptobjektiv 7 in die Objektebene 8 abgebildet. Bei dieser optischen Anordnung wird gleichzeitig die Wendel 20 der Lichtquelle 1 in die Pupille des Hauptobjektivs 7 abgebildet. Dadurch entsteht ein Wendelbild 20' auf der Netzhaut des Auges, dessen Reflexion in den stereoskopischen Beobachtungsstrahlengängen 13 und 14 sichtbar ist. Um diese und bereits beschriebene Nachteile auszuschließen, sind in diesem Ausführungsbeispiel das Kollektorlinsensystem 2 und die Leuchtfeldblende 3 auf einem Schieber 21 angeordnet, in dessen zweiter Schaltstellung eine weitere Lichtquelle 22 vorgesehen ist. Diese Lichtquelle 22 kann in den Beleuchtungsstrahlengang 19 an den Ort der Leuchtfeldblende 3 eingebracht und die Lichtquelle 1 deaktiviert werden. Bei dieser Anordnung wird dann die Lampenwendel der Lichtquelle 22 in die Objektebene 8 abgebildet, so daß kein scharfes Reflexbild an der Netzhaut mehr entstehen kann. Die spezifische Belastung der Netzhaut wird reduziert, da jetzt eine homogene Ausleuchtung vorliegt. Die Abbildung der Lampenwendel 20 der Lichtquelle 1 in die Objektebene 8 bewirkt darüber hinaus, daß das Objekt außerhalb der Pupille nicht beleuchtet wird, so daß der Kontrast des Red-Reflexes erhöht wird.

Die Figur 3 zeigt ein Ausführungsbeispiel, bei dem auf dem Schieber 21 das Kollektorlinsensystem 2 und in der anderen Schaltstellung eine Negativlinse 23 angeordnet ist. Bei feststehender Lichtquelle 1 und Leuchtfeldblende 3 wird der Abbildungsort der Lampenwendel wie nach der Beschreibung zur Figur 2 verändert.

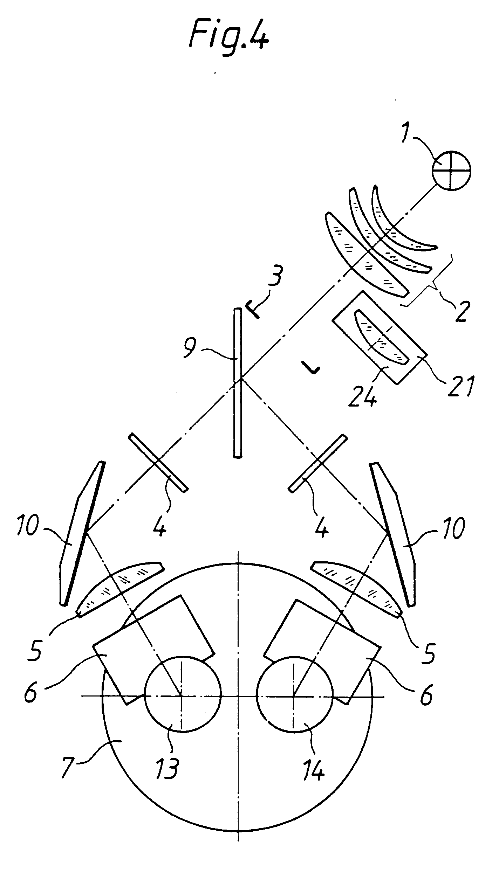

Die Figur 4 zeigt eine Beleuchtungsanordnung wie in der Figur 2, wobei hier das Kollektorlinsensystem 2 durch eine auf dem Schieber 21 angeordnete Positivlinse 24 zur Verlagerung des Wendelbildes ergänzt werden kann.

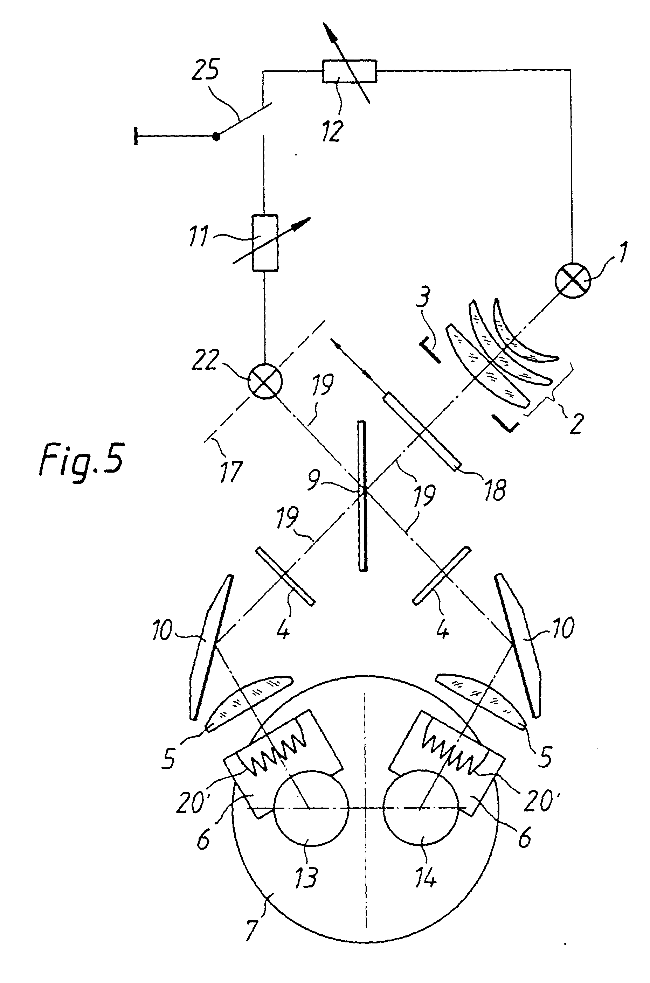

In der Figur 5 ist eine zusätzliche Lichtquelle 22 in einer zur Leuchtfeldblende 3 konjugierten Ebene 17 angeordnet. Das Licht dieser zusätzlichen Lichtquelle 22 wird über den Strahlenteiler 9 in die Beleuchtungsstrahlengänge 19 eingebracht. Zur Umschaltung sind die Lichtquellen 1 und 22 elektrisch mit einem Schalter 25 verbunden, so daß wahlweise eine der Lichtquellen 1 bzw. 22 aktiviert werden kann. Dieser Schalter 25 kann natürlich auch so ausgestaltet sein, daß sowohl die Lichtquelle 1 als auch die zusätzliche Lichtquelle 22 aktiviert sind. Diese Art der Mischbeleuchtung hat den Vorteil, daß neben dem verbesserten Red-Reflex auch eine Beleuchtung der Pupillenperipherie des Auges erreicht wird. Bei dieser Beleuchtungsart hat es sich als zweckmäßig erwiesen, wenn die Beleuchtungsstärke der Lichtquelle 1 reduziert wird. Dies kann beispielsweise dadurch erfolgen, daß ein Graufilter 18 in diesen Teil des Beleuchtungsstrahlenganges 19 eingebracht wird.

Im elektrischen Schaltkreis können auch Potentiometer 11, 12 vorgesehen sein, die die entsprechende Lampenspannung und damit die Helligkeit der Lichtquelle regeln.

Diese Anordnung mit einer zusätzlichen Lichtquelle 22 hat insbesondere den Vorteil, daß zur Umschaltung keine mechanischen bzw. optischen Veränderungen vorgenommen werden. Mit der Abbildung der Lampenwendel 20 in die Objektebene 8 wird erreicht, daß der Winkel zwischen der Beleuchtungsachse und der Beobachtungsachse bei Red-Reflex-Beleuchtung möglichst klein ist, während bei einer Köhler'schen Beleuchtung zur besseren plastischen Wahrnehmung des Objektes ein größerer Winkel realisiert wird.

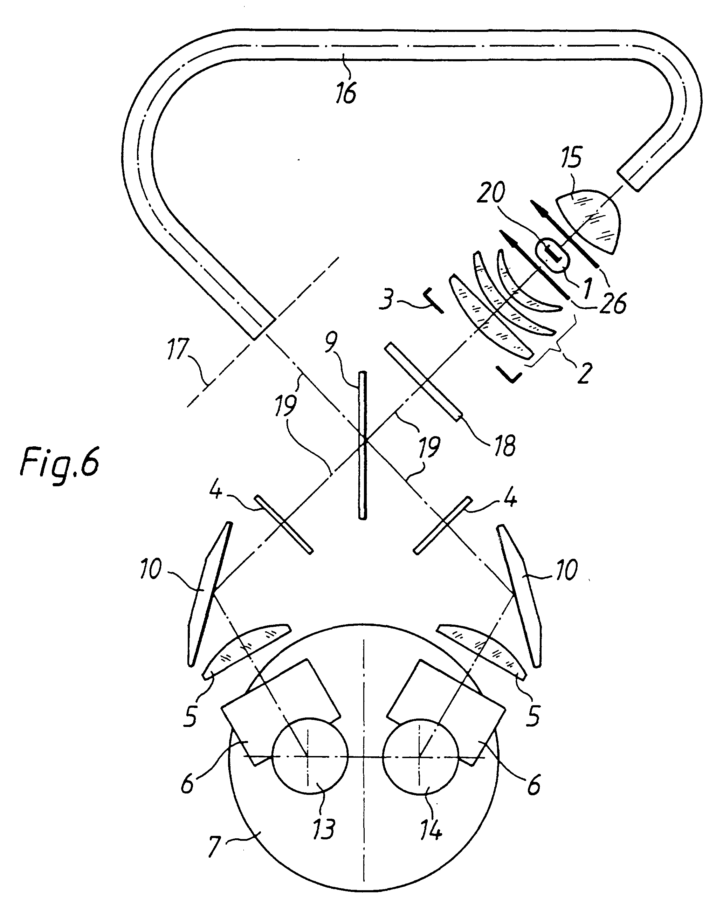

Die Figur 6 zeigt ein Ausführungsbeispiel der Erfindung mit einem weiteren Kollektor 15 und einem Lichtleiter 16. Die Lichteintrittsfläche des Lichtleiters 16 ist dem Kollektor 15 zugewandt und die Lichtaustrittsfläche in der zur Leuchtfeldblende 3 konjugierten Ebene 17 angeordnet. Zur wahlweisen Umschaltung der Beleuchtungsbedingungen sind zwei in den Beleuchtungsstrahlengang 19 einschiebbare Lichtstops 26 vorgesehen. Bei der Verwendung von Lichtleitern 16 kann mit der Wahl eines geeigneten Durchmessers in vorteilhafter Weise die Größe des Lichtfleckes auf dem Auge entsprechend angepaßt werden.

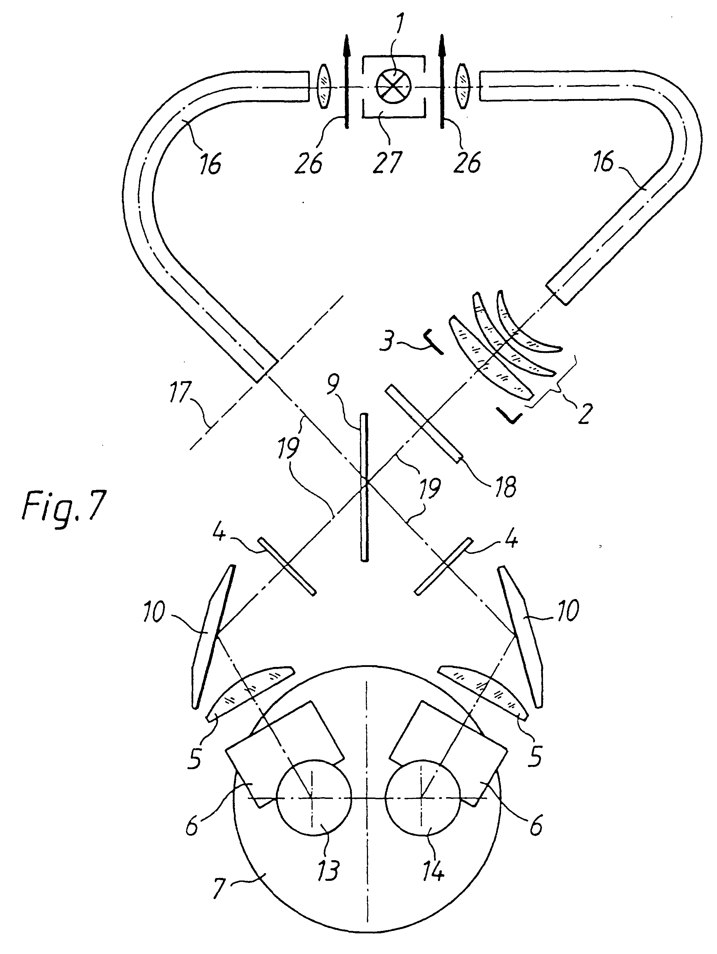

Die Figur 7 zeigt ein Ausführungsbeispiel wie in der Figur 6, jedoch mit einer extern und in einem separaten Gehäuse 27 zentral angeordneten Lichtquelle 1. An den jeweils gegenüberliegenden Seiten des Gehäuses sind die Lichtleiter 16 angeordnet. Zwischen dem Lampengehäuse 27 und den Lichteintrittsflächen der Lichtleiter 16 sind Lichtstops 26 zur wahlweisen Umschaltung oder Kombination der Beleuchtungsverhältnisse vorgesehen.

Um sowohl eine Red-Reflex-Beleuchtung als auch eine Köhler'sche gleichzeitig zu realisieren, wie es zum Ausführungsbeispiel gemäß der Figur 5 bzw. 6 bereits beschrieben wurde, können die Lichtstops 26 auch partiell in den Strahlengang eingebracht werden, um dadurch die Intensität der beiden Beleuchtungsverhältnisse entsprechend zu regeln.

Die Erfindung ist natürlich nicht auf die beschriebenen Ausführungsbeispiele beschränkt. Es liegt durchaus im Rahmen der Erfindung, die manuell beschriebenen Umschaltungen mit motorischen Mitteln auszustatten. Auch ist es denkbar, die elektrischen Schalter so auszubilden, daß neben einer wahlweisen Umschaltung auch ein gleichzeitiger Betrieb von zwei Lichtquellen ermöglicht wird.

### Bezugszeichenliste

- 1 -: Lichtquelle
- 2 -: Kollektorlinsensystem
- 3 -: Leuchtfeldblende
- 4 -: Filter
- 5 -: Linsen
- 6 -: Umlenkprisma
- 7 -: Hauptobjektiv
- 8 -: Objektebene
- 9 -: Strahlenteiler
- 10 -: Kaltlichtspiegel
- 11 -: Potentiometer
- 12 -: Potentiometer
- 13 -: Beobachtungsstrahlengang
- 14 -: Beobachtungsstrahlengang
- 15 -: Kollektor
- 16 -: Lichtleiter
- 17 -: zu 3 konjugierte Ebene
- 18 -: Filter
- 19 -: Beleuchtungsstrahlengang
- 20 -: Lampenwendel
- 20'-: Wendelbild
- 21 -: Schieber
- 22 -: zusätzliche Lichtquelle
- 23 -: Negativlinse
- 24 -: Postitivlinse
- 25 -: Schalter
- 26 -: Lichtstop
- 27 -: separates Lampenhaus

## Patentansprüche

1. Schaltbare Beleuchtungseinrichtung für ein Operationsmikroskop in der Augenchirurgie, mit mindestens einer Lichtquelle (1), einem Kollektorlinsensystem (2), einer Leuchtfeldblende (3), optischen Umlenkelementen (6,9,10), weiteren Linsen (5) und einem Hauptobjektiv (7), wobei das Beleuchtungslicht durch das Hauptobjektiv (7) in die Objektebene (8) gelenkt wird,
**dadurch gekennzeichnet,** daß die Leuchtfeldblende (3) über die Umlenkelemente (6,9,10) und die Linsen (5) durch das Hauptobjektiv (7) in die Objektebene (8) abgebildet wird und zur Erzeugung einer Red-Reflex-Beleuchtung schaltbare Mittel im Beleuchtungsstrahlengang vorgesehen sind, die das Bild der Leuchtfeldblende (3) verlagern und die Lampenwendel der Lichtquelle (1;22) in die Objektebene (8) abbilden.

2. Schaltbare Beleuchtungseinrichtung für ein Operationsmikroskop in der Augenchirurgie nach Anspruch 1, **dadurch gekennzeichnet, daß** die schaltbaren Mittel einen Schieber (21) aufweisen, auf dem das Kollektorlinsensystem (2), die Leuchtfeldblende (3) und eine zusätzliche Lichtquelle (22) angeordnet sind, wobei beim Umschalten das Kollektorlinsensystem (2) und die Leuchtfeldblende (3) aus dem Beleuchtungsstrahlengang (19) gebracht und die zusätzliche Lichtquelle (22) im Bereich der Leuchtfeldblende (3) eingebracht wird.

3. Schaltbare Beleuchtungseinrichtung für ein Operationsmikroskop in der Augenchirurgie nach Anspruch 1, **dadurch gekennzeichnet,** daß die schaltbaren Mittel einen Schieber (21) mit dem darauf angeordneten Kollektorlinsensystem (2) und einer Negativlinse (23) aufweisen, wobei beim Umschalten das Kollektorlinsensystem (2) gegen die Negativlinse (23) ausgetauscht wird.

4. Schaltbare Beleuchtungseinrichtung für ein Operationsmikroskop in der Augenchirurgie nach Anspruch 1, **dadurch gekennzeichnet,** daß die schaltbaren Mittel einen Schieber (21) mit einer darauf angeordneten Positivlinse (24) aufweisen derart, daß beim Umschalten diese Linse (24) zwischen dem Kollektorlinsensystem (2) und der Leuchtfeldblende (3) in den Beleuchtungsstrahlengang (19) eingebracht wird.

5. Schaltbare Beleuchtungseinrichtung für ein Operationsmikroskop in der Augenchirurgie, mit einer Lichtquelle (1), einem Kollektorlinsensystem (2), einer Leuchtfeldblende (3), optischen Umlenkelementen (6,9,10), weiteren Linsen (5) und einem Hauptobjektiv (7), wobei das Beleuchtungslicht durch das Hauptobjektiv (7) in die Objektebene (8) gelenkt wird,
**dadurch gekennzeichnet,** daß die Leuchtfeldblende (3) über die optischen Umlenkelemente (6,9,10) und die Linsen (5) durch das Hauptobjektiv (7) in die Objektebene (8) abgebildet wird, eines der optischen Umlenkelemente als Strahlenteiler (9) ausgebildet ist, und zur Erzeugung einer Red-Reflex-Beleuchtung über den Strahlenteiler (9) Beleuchtungslicht aus einem zusätzlichen Beleuchtungsstrahlengang in die Objektebene (8) gelenkt wird, wobei in dem zusätzlichen Beleuchtungsstrahlengang eine zusätzliche Lichtquelle (22) in einer zur Leuchtfeldblende (3) konjugierten Ebene (17) angeordnet ist und elektrische Mittel (25) zum Schalten der zusätzlichen Lichtquelle (22) vorgesehen sind.

6. Schaltbare Beleuchtungseinrichtung für ein Operationsmikroskop in der Augenchirurgie nach Anspruch 5, **dadurch gekennzeichnet,** daß über die elektrischen Mittel (25) die zusätzliche Lichtquelle (22) aktiviert und die Lichtquelle (1) deaktiviert wird.

7. Schaltbare Beleuchtungseinrichtung für ein Operationsmikroskop in der Augenchirurgie, mit einer Lichtquelle (1), einem Kollektorlinsensystem (2), einer Leuchtfeldblende (3), optischen Umlenkelementen (6,9,10), weiteren Linsen (5) und einem Hauptobjektiv (7), wobei das Beleuchtungslicht durch das Hauptobjektiv (7) in die Objektebene (8) gelenkt wird, **dadurch gekennzeichnet,** daß die Leuchtfeldblende (3) über die optischen Umlenkelemente (6,9,10) und die Linsen (5) durch das Hauptobjektiv (7) in die Objektebene (8) abgebildet wird, eines der optischen Umlenkelemente als Strahlenteiler (9) ausgebildet ist, und zur Erzeugung einer Red-Reflex-Beleuchtung über den Strahlenteiler (9) Beleuchtungslicht aus einem zusätzlichen Beleuchtungsstrahlengang in die Objektebene (8) gelenkt wird, im zusätzlichen Beleuchtungsstrahlengang ein Lichtleiter (16) angeordnet ist, dessen erstes Ende der Lichtquelle (1) und dessen gegenüberliegendes Ende in einer zur Leuchtfeldblende (3) konjugierten Ebene (17) angeordnet ist und zum Umschalten zwischen dem Kollektorlinsensystem (2) und dem Lichtleiter (16) ein in den Strahlengang (19) einbringbarer opaker Lichtstop (26) vorgesehen ist.

8. Schaltbare Beleuchtungseinrichtung für ein Operationsmikroskop in der Augenchirurgie nach Anspruch 7, **dadurch gekennzeichnet,** daß die Lichtquelle (1) in einem separaten Gehäuse (27) außerhalb des Mikroskops angeordnet ist, wobei im Beleuchtungsstrahlengang (19), der Lichtquelle (1) jeweils gegenüberliegend, je ein Lichtleiter (16) vorgesehen ist, einer der Lichtleiter (16) das Kollektorlinsensystem (2) beleuchtet und der andere Leiter (16) in der zur Leuchtfeldblende (3) konjugierten Ebene (17) endet, wobei zum Umschalten jeweils ein in den Beleuchtungsstrahlengang (19) einbringbarer opaker Lichtstop (26) vorgesehen ist.

9. Schaltbare Beleuchtungseinrichtung für ein Operations-mikroskop in der Augenchirurgie nach mindestens einem der Ansprüche 5 - 8, **dadurch gekennzeichnet,** daß sowohl die Leuchtfeldblende (3) als auch die Lampenwendel der Lichtquelle (1;22) über die Umlenkelemente (6,9,10) und die Linsen (5) durch das Hauptobjektiv (7) auf das zu betrachtende Patientenauge in die Objektebene (8) abgebildet werden.

10. Schaltbare Beleuchtungseinrichtung für ein Operationsmikroskop in der Augenchirurgie nach Anspruch 9, **dadurch gekennzeichnet,** daß im Beleuchtungsstrahlengang (19) zwischen dem Kollektorlinsensystem (2) und dem Strahlenteiler (9) ein Filter (18) angeordnet ist, der die durchgelassene Lichtintensität reduziert.

11. Schaltbare Beleuchtungseinrichtung für ein Operationsmikroskop in der Augenchirurgie nach mindestens einem der vorherigen Ansprüche, **dadurch gekennzeichnet,** daß im elektrischen Schaltkreis der Lichtquelle (1;22) ein Potentiometer (11;12) vorgesehen ist, über das die Lampenspannung und damit die Helligkeit der zugehörigen Lichtquelle (1;22) unabhängig regelbar ist.

## Claims

1. Switchable illumination equipment for a surgical microscope for eye surgery with at least one light source (1), a collector lens system (2), a light field stop (3), optical deflecting elements (6, 9, 10), further lenses (5) and a main objective (7), wherein the illuminating light is deflected through the main objective (7) into the object plane (8), characterised in that the light field stop (3) is imaged by way of the deflecting elements (6, 9, 10) and the lenses (5) into the object plane (8) and switchable means, which redispose the image of the light field stop (3) and image the lamp filaments of the light source (1; 22) into the object plane (8), are provided in the illumination ray path for the production of a red reflex illumination.

2. Switchable illumination equipment for a surgical microscope for eye surgery according to claim 1, characterised in that the switchable means comprise a slide (21), on which the collector lens system (2), the light field stop (3) and an additional light source (22) are arranged, wherein the collector lens system (2) and the light field stop (3) are brought out of the illumination ray path (19) during the switching-over and the additional light source (22) is introduced in the region of the light field stop (3).

3. Switchable illumination equipment for a surgical microscope for eye surgery according to claim 1, characterised in that the switchable means comprise a slide (21) with the collector lens system (2) mounted thereon and a negative lens (23), wherein the collector lens system (2) is exchanged for the negative lens (23) during the switching-over.

4. Switchable illumination equipment for a surgical microscope for eye surgery according to claim 1, characterised in that the switchable means comprise a slide (21) with a positive lens (24) mounted thereon in such a manner that this lens (24) is introduced into the illumination ray path (19) between the collector lens system (2) and the light field stop (3) during the switching-over.

5. Switchable illumination equipment for a surgical microscope for eye surgery with a light source (1), a collector lens system (2), a light field stop (3), optical deflecting elements (6, 9, 10), further lenses (5) and a main objective (7), wherein the illuminating light is deflected through the main objective (7) into the object plane (8), characterised in that the light field stop (3) is imaged through the main objective (7) by way of the deflecting elements (6, 9, 10) and the lenses (5) into the object plane (8), one of the optical deflecting elements is constructed as beam splitter (9) and illumination light out of an additional illumination ray path is deflected by way of the beam splitter (9) into the object plane (8) for the production of a red reflex illumination, wherein an additional light source (22) is arranged in the additional illumination ray path in a plane (17) conjugate with the light field stop (3) and electrical means (25) are provided for the switching of the additional light source (22).

6. Switchable illumination equipment for a surgical microscope for eye surgery according to claim 5, characterised in that the additional light source (22) is activated and the light source (1) is deactivated by way of the electrical means (25).

7. Switchable illumination equipment for a surgical microscope for eye surgery with a light source (1), a collector lens system (2), a light field stop (3), optical deflecting elements (6, 9, 10), further lenses (5) and a main objective (7), wherein the illuminating light is deflected through the main objective (7) into the object plane (8), characterised in that the light field stop (3) is imaged through the main objective (7) by way of the deflecting elements (6, 9, 10) and the lenses (5) into the object plane (8), one of the optical deflecting elements is constructed as beam splitter (9) and illumination light out of an additional illumination ray path is deflected by way of the beam splitter (9) into the object plane (8) for the production of a red reflex illumination, an optical conductor (16), the first end of which faces the light source (1) and the opposite end of which is arranged in a plane (17) conjugate with the light field stop (3), is arranged in the additional illumination ray path, and an opaque light stop (26), which is introducible into the ray path (19), is provided for the switching-over between the collector lens system (2) and the optical conductor (16).

8. Switchable illumination equipment for a surgical microscope for eye surgery according to claim 7, characterised in that the light source (1) is arranged in a separate housing (27) outside the microscope, wherein a respective optical conductor (16) each lying opposite the light source (1), is provided in the illumination ray path (19), one of the optical conductors (16) illuminating the collector lens system (2) and the other conductor (16) ending in the plane (17) conjugate with the light field stop (3), wherein a respective opaque light stop (26), which is introducible into the illumination ray path (19), is provided for the switching-over.

9. Switchable illumination equipment for a surgical microscope for eye surgery according to at least one of the claims 5 to 8, characterised in that the light field stop (3) as well as also the lamp filaments of the light source (1; 22) are imaged by way of the deflecting elements (6, 9, 10) and the lenses (5) through the main objective (7) onto the patient's eye to be observed in the object plane (8).

10. Switchable illumination equipment for a surgical microscope for eye surgery according to claim 9, characterised in that a filter (18), which reduces the transmitted light intensity, is arranged in the illumination ray path (19) between the collector lens system (2) and the beam splitter (9).

11. Switchable illumination equipment for a surgical microscope for eye surgery according to at least one of the preceding claims, characterised in that a potentiometer (11; 12), by way of which the lamp voltage and thereby the brightness of the associated light source (1; 12) can be regulated independently, is provided in the electrical switching circuit of the light source (1; 12).

## Revendications

1. Dispositif d'éclairage commutable pour un microscope chirurgical dans la chirurgie des yeux avec au moins une source de lumière (1), un système de lentilles de collecteur (2), un obturateur du champ lumineux (3), des éléments optiques de déviation (6, 9, 10), d'autres lentilles (5) et un objectif principal (7), où la lumière d'éclairage est déviée à travers l'objectif principal (7) dans le plan de l'objet (8),
caractérisé en ce que l'obturateur du champ lumineux (3) est représenté par les éléments de déviation (6, 9 10) et les lentilles (5) à travers l'objectif principal (7) dans le plan (8) de l'objet et pour la production d'un éclairage à reflet rouge, des moyens commutables sont prévus dans la trajectoire des rayons d'éclairage, qui déplacent l'image de l'obturateur du champ lumineux (3) et représentent le filament de lampe de la source de lumière (1 ; 22) dans le plan (8) de l'objet.

2. Dispositif d'éclairage commutable pour un microscope chirurgical dans la chirurgie des yeux selon la revendication 1, caractérisé en ce que les moyens commutables présentent un coulisseau (21) sur lequel sont agencés le système de lentilles de collecteur (2), l'obturateur du champ lumineux (3) et une source de lumière supplémentaire (22), et par commutation, le système de lentilles de collecteur (2) et l'obturateur du champ lumineux (3) sont portés hors de la trajectoire des rayons d'éclairage (19) et la source de lumière supplémentaire (22) est introduite dans la zone de l'obturateur du champ lumineux (3).

3. Dispositif d'éclairage commutable pour un microscope chirurgical dans la chirurgie des yeux selon la revendication 1, caractérisé en ce que les moyens commutables présentent un coulisseau (21) avec le système de lentilles de collecteur (2) qui y est agencé et une lentille négative (23), et lors de la commutation, le système de lentilles de collecteur (2) est échangé pour la lentille négative (23).

4. Dispositif d'éclairage commutable pour un microscope chirurgical dans la chirurgie des yeux selon la revendication 1, caractérisé en ce que les moyens commutables présentent un coulisseau (21) avec une lentille positive (24) qui y est agencée de façon que lors de la commutation cette lentille (24) soit introduite entre le système de lentilles de collecteur (2) et l'obturateur du champ lumineux (3) dans la trajectoire des rayons d'éclairage.

5. Dispositif d'éclairage commutable pour un microscope chirurgical dans la chirurgie des yeux avec une source de lumière (1), un système de lentilles de collecteur (2), un obturateur du champ lumineux (3), des éléments optiques de déviation (6, 9, 10), d'autres lentilles (5) et un objectif principal (7), où la lumière d'éclairage est déviée à travers l'objectif principal (7) dans le plan de l'objet (8),
caractérisé en ce que l'obturateur du champ lumineux (3) est représenté par les éléments optiques de déviation (6, 9, 10) et les lentilles (5) à travers l'objectif principal (7) dans le plan de l'objet (8), l'un des éléments optiques de déviation est configuré en diviseur de rayons (9) et pour la production d'un éclairage à reflet rouge, la lumière d'éclairage est déviée par le diviseur de rayons (9) à partir d'une trajectoire supplémentaire des rayons d'éclairage dans le plan de l'objet, et dans la trajectoire des rayons d'éclairage supplémentaire, est agencée une source de lumière supplémentaire (22) dans un plan (17) conjugué à l'obturateur du champ lumineux (3) et des moyens électriques (25) sont prévus pour la commutation de la source supplémentaire de lumière (22).

6. Dispositif d'éclairage commutable pour un microscope chirurgical dans la chirurgie des yeux selon la revendication 5, caractérisé en ce que par les moyens électriques (25), la source supplémentaire de lumière (22) est activée et la source de lumière (1) est désactivée.

7. Dispositif d'éclairage commutable pour un microscope chirurgical dans la chirurgie des yeux avec une source de lumière (1), un système de lentilles de collecteur (2), un obturateur du champ lumineux (3), des éléments optiques de déviation (6, 9, 10), d'autres lentilles (5) et un objectif principal (7), où la lumière d'éclairage est déviée par l'objectif principal (7) dans le plan de l'objet (8), caractérisé en ce que l'obturateur du champ lumineux (3) est représenté par les éléments optiques de déviation (6, 9, 10) et les lentilles (5) à travers l'objectif principal (7) dans le plan de l'objet (8), l'un des éléments optiques de déviation est formé d'un diviseur de rayons (9) et, pour la production d'un éclairage à reflet rouge, la lumière d'éclairage d'une trajectoire supplémentaire des rayons d'éclairage est déviée dans le plan de l'objet (8), dans la trajectoire supplémentaire des rayons d'éclairage est agencé un conducteur de lumière (16), dont la première extrémité est agencée dans la source de lumière (1) et dont l'extrémité opposée est agencée dans un plan (17) conjugué à l'obturateur du champ lumineux (3) et pour la commutation entre le système de lentilles de collecteur (2) et le conducteur de lumière (16), est prévue une butée de lumière (26) opaque pouvant être introduite dans la trajectoire des rayons (19).

8. Dispositif d'éclairage commutable pour un microscope chirurgical dans la chirurgie des yeux selon la revendication 7, caractérisé en ce que la source de lumière (1) est agencée dans un boîtier séparé (27) en dehors du microscope, et dans la trajectoire des rayons d'éclairage (19), est prévu à chaque fois un conducteur de lumière (16), face à la source de lumière (1), l'un des conducteurs de lumière (16) éclaire le système de lentilles de collecteur (2) et l'autre conducteur (16) se termine dans le plan (17) conjugué à l'obturateur du champ lumineux (3) et pour la commutation est prévue à chaque fois une butée de lumière opaque (26) pouvant être introduite dans la trajectoire des rayons d'éclairage (19).

9. Dispositif d'éclairage commutable pour un microscope chirurgical dans la chirurgie des yeux selon au moins l'une des revendications 5-8, caractérisé en ce qu'aussi bien l'obturateur du champ lumineux (3) qu'également le filament de lampe de la source de lumière (1 ; 22) sont représentés par les éléments de déviation (6, 9, 10) et les lentilles (5) à travers l'objectif principal (7) sur l'oeil de patient à observer dans le plan (8) de l'objet.

10. Dispositif d'éclairage commutable pour un microscope chirurgical en chirurgie des yeux selon la revendication 9, caractérisé en ce que dans la trajectoire des rayons d'éclairage (19), entre le système de lentilles de collecteur (2) et le diviseur de rayons (9) est agencé un filtre (18) qui réduit l'intensité de la lumière qui passe.

11. Dispositif d'éclairage commutable pour un microscope chirurgical dans la chirurgie des yeux selon au moins l'une des revendications précédentes, caractérisé en ce que dans le circuit électrique de la source de lumière (1 ; 22) est prévu un potentiomètre (11 ; 12) par lequel la tension de la lampe et ainsi la clarté de la source de lumière correspondante (1 ; 22) est réglable indépendamment.
